(19) Europäisches Patentamt
European Patent Office
Office européen des brevets

(11) EP 0 686 389 B1

(12) FASCICULE DE BREVET EUROPEEN

(45) Date de publication et mention de la délivrance du brevet:
**04.09.1996 Bulletin 1996/36**

(51) Int. Cl.$^6$: **A61K 7/13**

(21) Numéro de dépôt: **95400980.9**

(22) Date de dépôt: **28.04.1995**

# (54) Composition de teinture d'oxydation des fibres kératiniques comprenant la 2-(B-hydroxyéthyl) paraphénylènediamine, la 2-méthylrésorcine et le 3-aminophénol, et procédé de teinture utilisant une telle composition

Zusammensetzung zum Färben von Keratinfasern, enthaltend 2-(beta-Hydroxyethyl)paraphenylendiamin, 2-Methylresorcin und 3-Aminophenol, und Verfahren zur Anwendung

Composition for dying keratinous fibres comprising 2-(B-hydroxyéthyl)paraphenylene diamine, 2-methylresorcin and 3-aminophenol, and method for using it

(84) Etats contractants désignés:
**DE ES FR GB IT**

(30) Priorité: **06.06.1994 FR 9406900**

(43) Date de publication de la demande:
**13.12.1995 Bulletin 1995/50**

(73) Titulaire: **L'OREAL**
**F-75008 Paris (FR)**

(72) Inventeurs:
- **Cotteret, Jean**
**F-78480 Verneuil sur Seine (FR)**
- **Audousset, Marie-Pascale**
**F-92600 Asnières (FR)**

(74) Mandataire: **Andral, Christophe André Louis**
**L'OREAL**
**Centre de Recherche Charles Zviak**
**Département Propriété Industrielle**
**90, rue du Général Roguet**
**92583 Clichy Cedex (FR)**

(56) Documents cités:

| | |
|---|---|
| **EP-A- 0 400 330** | **WO-A-80/00214** |
| **WO-A-88/00823** | **DE-A- 3 706 565** |
| **DE-A- 3 834 143** | **GB-A- 2 168 727** |

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Rank Xerox (UK) Business Services
2.13.4/3.4

## Description

La présente invention concerne une composition de teinture d'oxydation des fibres kératiniques et en particulier des fibres kératiniques humaines, comprenant en association, de la 2-β-hydroxyéthyl paraphénylènediamine, de la 2-méthyl résorcine et du 3-amino phénol. Elle concerne également l'utilisation d'une telle composition.

Il est connu de teindre les fibres kératiniques, et en particulier les cheveux humains, avec des compositions de teinture contenant des précurseurs de colorants d'oxydation, en particulier des ortho- ou paraphénylènediamines, des ortho-ou paraaminophénols, généralement appelés "bases d'oxydation", associés à des coupleurs encore appelés modificateurs de coloration, plus particulièrement des métaphénylènediamines, des méta-aminophénols et des métadiphénols, qui permettent de modifier et d'enrichir en reflets les colorations "de fond" obtenues par les produits de condensation des bases d'oxydation.

On recherche, dans le domaine de la teinture capillaire d'oxydation, des précurseurs de colorants d'oxydation et des coupleurs capables d'engendrer, lorsqu'ils sont associés, des nuances ayant une résistance satisfaisante à la lumière, aux lavages, aux intempéries, à la transpiration et aux différents traitements capillaires que peuvent subir les cheveux.

Jusqu'ici ces nuances ont été obtenues avec des teintures à base de paraphénylènediamine. Mais actuellement l'utilisation de la paraphénylènediamine est remise en question pour des raisons toxicologiques.

En remplacement de la paraphénylènediamine, on a déjà proposé, dans la demande de brevet WO 80/00214, d'utiliser des dérivés de la paraphénylènediamine monohydroxyalkylés en position 2 sur le noyau benzénique.

Dans le brevet EP-0 400 330 B1, on a décrit tout particulièrement une association de la 2-β-hydroxyéthyl paraphénylènediamine, avec un coupleur choisi parmi la résorcine, la 2-méthyl résorcine, la 4-chloro résorcine, le 3,4-méthylènedioxy phénol, le 3-amino phénol et la N-(2-hydroxyéthyl) 3,4-méthylènedioxy aniline. L'exemple 2 de ce document illustre notamment l'association de la 2-β-hydroxyéthyl paraphénylènediamine avec la résorcine et le 3-amino phénol.

Cette association de l'exemple 2 conduit cependant à une nuance peu résistante à la transpiration, ce qui se traduit par un virage important de la nuance après que les cheveux aient subi l'action de la transpiration.

Or, après d'importantes recherches menées sur la question, la Demanderesse vient maintenant de découvrir qu'il est possible d'obtenir de nouvelles teintures non toxiques, qui engendrent des nuances particulièrement bien résistantes à la transpiration, en associant la 2-β-hydroxyéthyl paraphénylènediamine à la 2-méthyl résorcine et au 3-amino phénol.

Cette découverte est à la base de la présente invention.

La présente invention a ainsi pour objet une composition de teinture d'oxydation pour fibres kératiniques, en particulier pour fibres kératiniques humaines telles que les cheveux, du type comprenant, dans un milieu approprié pour la teinture, au moins un précurseur de colorant d'oxydation et au moins un coupleur, et qui est caractérisée par le fait qu'elle contient, à titre de précurseur de colorant d'oxydation, de la 2-β-hydroxyéthyl paraphénylènediamine, et à titre de coupleur, une association de la 2-méthyl résorcine et du 3-amino phénol, ou les sels d'addition de ces différents composés avec un acide.

Les nouvelles teintures ainsi obtenues permettent d'aboutir à des nuances bien plus résistantes à la transpiration que celles de l'art antérieur renfermant au moins la 2-β-hydroxyéthyl paraphénylènediamine associée à la résorcine et au 3-amino phénol.

En outre, ces nouvelles teintures, lorsqu'elles sont appliquées notamment sur des cheveux qui ont été sensibilisés par une déformation permanente, se sont révélées également beaucoup plus résistantes aux shampooings que les teintures de l'art antérieur associant de la 2-β-hydroxyéthyl paraphénylènediamine avec la 2-méthyl résorcine, ou de la 2-β-hydroxyéthyl paraphénylènediamine avec le 3-amino phénol.

Un autre objet de l'invention porte sur la composition prête à l'emploi, contenant les différents agents utilisés pour la teinture des fibres kératiniques définis ci-dessus et un agent oxydant.

L'invention vise également un procédé de teinture des fibres kératiniques, et en particulier des fibres kératiniques humaines telles que les cheveux, consistant à appliquer sur ces fibres au moins une composition (A) contenant, dans un milieu approprié pour la teinture, au moins un précurseur de colorant d'oxydation et au moins deux coupleurs tels qu'ils ont été définis ci-avant, la couleur étant révélée à pH alcalin neutre ou acide à l'aide d'un agent oxydant qui est ajouté juste au moment de l'emploi à la composition (A) ou qui est présent dans une composition (B) appliquée simultanément ou séquentiellement de façon séparée.

L'invention a également pour objet des dispositifs de teinture ou "kits" à plusieurs compartiments, dont le premier compartiment contient au moins la 2-β-hydroxyéthyl paraphénylènediamine à titre de précurseur de colorant d'oxydation, et au moins l'association de la 2-méthyl résorcine et du 3-amino phénol à titre de coupleurs, et le deuxième compartiment un agent oxydant.

D'autres caractéristiques, aspects, objets et avantages de l'invention apparaîtront encore plus clairement à la lecture de la description et des exemples qui suivent.

Les sels d'acide qui peuvent être utilisés selon l'invention sont choisis de préférence parmi les chlorhydrates, les sulfates, les bromhydrates et les tartrates.

La concentration en précurseur de colorant d'oxydation, ou en ses sels, peut varier de préférence entre 0,01 et 10 % en poids environ par rapport au poids total de la composition de teinture, et encore plus préférentiellement entre 0,05 et 5 % en poids environ.

La concentration en 2-méthyl résorcine ou en ses sels peut varier de préférence entre 0,005 et 5% en poids environ par rapport au poids total de la composition de teinture, et encore plus préférentiellement entre 0,05 et 3 % en poids environ.

La concentration en 3-amino phénol ou en ses sels peut varier de préférence entre 0,005 et 5% en poids environ par rapport au poids total de la composition de teinture, et encore plus préférentiellement entre 0,05 et 3 % en poids environ.

L'agent oxydant est de préférence choisi parmi le peroxyde d'hydrogène, le peroxyde d'urée, les bromates de métaux alcalins, les persels tels que les perborates et les persulfates. L'utilisation du peroxyde d'hydrogène est particulièrement préférée.

La composition (A), qui renferme l'association des colorants telle que décrite ci-dessus, peut avoir un pH compris entre 3 et 11, qui peut être ajusté à la valeur choisie soit au moyen d'agents alcalinisants habituellement utilisés en teinture des fibres kératiniques, tels que l'ammoniaque, les carbonates alcalins, les alcanolamines, par exemple les mono-, di- et triéthanolamines ainsi que leurs dérivés, les hydroxydes de sodium ou de potassium, les composés de formule :

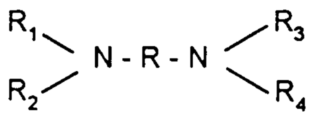

dans laquelle R est un reste propylène éventuellement substitué par un groupement hydroxyle ou un radical alkyle en $C_1$-$C_4$ ; $R_1$ $R_2$, $R_3$ et $R_4$, simultanément ou indépendamment l'un de l'autre, représentent un atome d'hydrogène, un radical alkyle en $C_1$-$C_4$ ou hydroxyalkyle en $C_1$-$C_4$,
soit au moyen d'agents acidifiants classiques, tels que les acides minéraux ou organiques comme par exemple les acides chlorhydrique, tartrique, citrique et phosphorique.

Le pH de la composition (B) renfermant l'agent oxydant tel que défini ci-dessus, est tel qu'après mélange avec la composition (A), le pH de la composition appliquée sur les fibres kératiniques humaines varie de préférence entre 3 et 11. Il est ajusté à la valeur désirée à l'aide d'agents acidifiants ou éventuellement alcalinisants bien connus de l'état de la technique, tels que ceux décrits ci-dessus.

La composition oxydante (B) est de préférence constituée par une solution d'eau oxygénée.

Selon un mode de réalisation préféré du procédé de teinture de l'invention, on mélange, au moment de l'emploi, la composition de teinture (A) décrite ci-dessus avec une solution oxydante en une quantité suffisante pour développer une coloration. Le mélange obtenu est ensuite appliqué sur les fibres kératiniques humaines et on laisse poser pendant 5 à 40 minutes, de préférence 15 à 30 minutes, après quoi on rince, on lave au shampooing, on rince à nouveau et on sèche.

Les compositions de teinture peuvent encore contenir, en plus des colorants définis ci-dessus, d'autres coupleurs et/ou des colorants directs, notamment pour modifier les nuances ou les enrichir en reflets.

Les compositions de teinture peuvent également contenir des agents antioxydants. Ceux-ci peuvent être choisis en particulier parmi le sulfite de sodium, l'acide thioglycolique, l'acide thiolactique, le bisulfite de sodium, l'acide déhydroascorbique, l'hydroquinone, la 2-méthyl hydroquinone, la tert.butyl hydroquinone et l'acide homogentisique et sont alors généralement présents dans des proportions comprises entre environ 0,05 et 1,5 % en poids par rapport au poids total de la composition.

Les compositions de teinture contiennent également, dans leur forme de réalisation préférée, des agents tensioactifs bien connus de la technique, dans des proportions comprises entre environ 0,5 et 55 % en poids, et de préférence entre 2 et 50 % en poids, par rapport au poids total de la composition, des solvants organiques, dans des proportions comprises entre environ 1 et 40 % en poids, et en particulier entre 5 et 30 % en poids par rapport au poids total de la composition, ou tout autre adjuvant cosmétiquement acceptable et connu de la technique antérieure en teinture d'oxydation capillaire.

La composition appliquée sur les cheveux peut se présenter sous des formes diverses, telles que sous forme de liquide, de crème, de gel, ou sous toute autre forme appropriée pour réaliser une teinture des fibres kératiniques, et notamment des cheveux humains. En particulier, elle peut être conditionnée sous pression en flacon aérosol en présence d'un agent propulseur et former une mousse.

Des exemples concrets illustrant l'invention vont maintenant être donnés. On commencera par définir les tests utilisés pour évaluer les performances des teintures d'oxydation selon l'invention, concernant leur résistance à la transpiration, et au shampooing.

Résistance à la transpiration :

On utilise une solution de sueur synthétique de composition suivante :

| | |
|---|---|
| - NaCl | 1 g |
| - Hydrogénophosphate de potassium | 0,1 g |
| - Histidine | 0,025 g |
| - Acide lactique q.s. | pH 3,2 |
| - Eau distillée q.s.p. | 100 g |

Dans un cristallisoir recouvert d'un verre de montre et renfermant cette solution de sueur, on immerge les mèches de cheveux teints qu'on laisse séjourner de 20 à 50 heures à 37° C. On rince ensuite les mèches et on les sèche.

Résistance au shampooing (machine Ahiba-Texomat) :

On place des mèches de cheveux teints dans un panier que l'on immerge dans une solution d'un shampooing standard. Le panier est soumis à un mouvement de va-et-vient vertical de fréquence variable ainsi qu'à un mouvement de rotation qui reproduisent l'action d'un frottement manuel, ce qui engendre la formation de mousse.

Après 3 minutes d'épreuve, on retire les mèches que l'on rince puis sèche.

**EXEMPLES**

**EXEMPLE 1**

On a préparé la composition de teinture, conforme à l'invention, suivante :

| | |
|---|---|
| - 2-β-hydroxyéthyl paraphénylènediamine, sulfate | 3,0 g |
| - 2-méthyl résorcine | 0,60 g |
| - 3-amino phénol | 0,6 g |
| - Alcool cétylique | 15,0 g |
| - Sulfite de sodium | 0,3g |
| - Lauryléthersulfate de sodium en solution aqueuse à 28 % de matière active (M.A.) | 0,98 g M.A. |
| - Ammoniaque à 20 % de $NH_3$ | 0,75 g M.A. |
| - Eau déminéralisée q.s.p. | 100,0 g |

Au moment de l'emploi, on a mélangé cette composition poids pour poids avec de l'eau oxygénée titrant 20 volumes (6% en poids), de pH 3.

On a obtenu un mélange de pH 9,8.

On a ensuite appliqué ce mélange sur des cheveux gris naturels à 90% de blancs, pendant 30 minutes et à 40°C. Après rinçage, lavage au shampooing, rinçage et séchage, les cheveux sont teints et l'on soumet alors ces cheveux teints au test de résistance à la transpiration (48 heures) décrit ci-dessus.

Parallèlement, et suivant le même procédé que celui décrit ci-avant, on teint des cheveux à l'aide d'une composition de teinture de l'art antérieur (exemple 2 du brevet EP 0 400 330 B1), c'est à dire un contretype de la composition selon l'invention décrite ci-dessus dans laquelle on a remplacé la 2-méthylrésorcine par une quantité pondérale identique de résorcine. On soumet les cheveux teints à l'aide de la composition de teinture contretype au test de résistance à la transpiration (48 heures) dans les mêmes conditions que les cheveux teints à l'aide de la composition selon l'invention.

Les nuances sont mesurées au MINOLTA CM 2002, et chiffrées en valeurs MUNSELL (Norme ASTM D 1535-68).

Les nuances des cheveux teints, chiffrées en Hue (H) avant puis après qu'ils aient subi le test de résistance à la transpiration, sont réunies dans le tableau ci-après, avec indication de l'écart de nuance en ΔH :

| Valeur de la HUE | **avant** l'épreuve de la transpiration | **après** l'épreuve de la transpiration | **ΔH** |
|---|---|---|---|
| Composition selon l'invention | 5,6 YR | 4,3 YR | 1,3 |
| Contretype à la résorcine | 4,1 R | 3,9 YR | 9,8 |

On constate donc bien, de façon inattendue et surprenante, un virage nettement moins important de la nuance pour les cheveux teints avec la composition selon l'invention, (ΔH = 1,3) comparativement aux cheveux teints avec la composition contretype (ΔH = 9,8).

**EXEMPLE 2**

On a préparé la composition de teinture, conforme à l'invention, suivante :

| | |
|---|---|
| - 2-β-hydroxyéthyl paraphénylènediamine, dichlorhydrate | 0,60 g |
| - 2-méthyl résorcine | 0,20 g |
| - 3-amino phénol | 0,20 g |
| - Alcool oléique polyglycérolé à 4 moles de glycérol, à 78 % de matières actives (M.A.) | 5,7 g M.A. |
| - Alcool oléique polyglycérolé à 2 moles de glycérol | 4,0 g |
| - Acide oléique | 3,0 g |
| - Amine oléique à 2 moles d'oxyde d'éthylène, vendue sous la dénomination Ethomeen 012 par la Société AKZO | 7,0 g |
| - Laurylamino succinamate de diéthylaminopropyle, sel de sodium, à 55 % de M.A. | 3,0 g M.A. |
| - Alcool oléique | 5,0 g |
| - Diéthanolamide d'acide oléique | 12,0 g |
| - Propylèneglycol | 3,5 g |
| - Alcool éthylique | 7,0 g |
| - Dipropylèneglycol | 0,5 g |
| - Monométhyléther de propylèneglycol | 9,0 g |
| - Métabisulfite de sodium en solution aqueuse à 35 % de M.A. | 0,46 g M.A. |
| - Acétate d'ammonium | 0,8 g |
| - Antioxydant, séquestrant, | q.s. |
| - Parfum, conservateur, | q.s. |
| - Solution aqueuse d'ammoniaque à 20 % de $NH_3$ | 2,0 g M.A. |
| - Eau déminéralisée q.s.p. | 100,0 g |

Au moment de l'emploi, on a mélangé cette composition poids pour poids avec de l'eau oxygénée titrant 20 volumes (6% en poids), de pH 3.

On a obtenu un mélange de pH 9,8.

On applique ce mélange sur des cheveux gris permanentés à 90 % de blancs, pendant 30 minutes. Après rinçage, lavage au shampooing, rinçage et séchage, les cheveux sont teints; on les soumet alors au test de résistance au shampooing décrit ci-avant.

On réalise, parallèlement, deux compositions comparatives qui renferment, en remplacement de l'association selon l'invention constituée du dichlorhydrate de 2-β-hydroxyéthyl paraphénylènediamine + 2-méthyl résorcine + 3-amino phénol, les associations suivantes :

*Composition comparative (A)* :
2-β-hydroxyéthyl paraphénylènediamine, dichlorhydrate (0,6 g) + 2-méthyl résorcine (0,4 g),

*Composition comparative (B)* :
2-βhydroxyéthyl paraphénylènediamine, dichlorhydrate (0,6 g) + 3-amino phénol (0,4 g).

***Evaluation de la résistance au shampooing :***

On exprime la dégradation de la couleur entre les cheveux teints et ceux teints ayant subi une épreuve shampooing telle que décrite ci-avant, au moyen de l'équation de NICKERSON qui définit les indices de variation de couleur :

$$\Delta E = 0{,}4\ C_0\ \Delta H + 6\Delta V + 3\Delta C;$$

cette équation est décrite dans la publication : "Journal of the Optical Society of America ", 1944 - sept - Vol. 34 - n°9 - p 550-570, les paramètres H, V, et C, représentant les paramètres de la notation MUNSELL (Norme ASTM D 1535-68), laquelle définit la couleur, (H désignant la nuance ou HUE, V désignant l'intensité ou VALUE, C désignant la pureté ou CHROMATICITE et $C_0$ désignant la pureté de la mèche par rapport à laquelle on désire évaluer la différence de couleur).

Les dégradations de la couleur enregistrées avec la composition selon l'invention et celles des compositions comparatives (A) et (B) sont réunies dans le tableau suivant :

| Composition de teinture | Variation de couleur entre cheveux teints et cheveux teints ayant subi une épreuve shampooing (ΔE) |
|---|---|
| Composition de l'invention | 1,66 |
| Composition comparative (A) | 4,15 |
| Composition comparative (B) | 8,58 |

La dégradation de couleur étant d'autant plus importante que le chiffre indiqué (ΔE) est élevé, on constate donc bien, de façon inattendue et surprenante, une résistance au shampooing bien supérieure avec la composition de teinture selon l'invention.

**Revendications**

1. Composition de teinture d'oxydation pour fibres kératiniques, en particulier pour fibres kératiniques humaines telles que les cheveux, du type comprenant, dans un milieu approprié pour la teinture, au moins un précurseur de colorant d'oxydation et au moins un coupleur, caractérisée par le fait qu'elle contient, à titre de précurseur de colorant d'oxydation, de la 2-β-hydroxyéthyl paraphénylènediamine et à titre de coupleur, une association de 2-méthyl résorcine et de 3-amino phénol, ou les sels d'addition de ces différents composés avec un acide.

2. Composition de teinture selon la revendication 1, caractérisée par le fait que les sels d'addition avec un acide sont choisis parmi les chlorhydrates, les sulfates, les bromhydrates et les tartrates.

3. Composition de teinture selon l'une des revendications 1 à 2, caractérisée par le fait que la 2-β-hydroxyéthyl paraphénylènediamine ou ses sels est présente dans une concentration comprise entre 0,01 et 10 % en poids par rapport au poids total de la composition, de préférence entre 0,05 et 5 % en poids, que la 2-méthyl résorcine ou ses sels est présente dans une concentration comprise entre 0,01 et 5 % en poids par rapport au poids total de la composition, de préférence entre 0,05 et 3 % en poids et que le 3-aminophénol ou ses sels est présent dans une concentration comprise entre 0,01 et 5% en poids par rapport au poids total de la composition, de préférence entre 0,05 et 3% en poids.

4. Composition de teinture selon l'une quelconque des revendications 1 à 3, prête à l'emploi, caractérisée en ce qu'elle contient en outre un agent oxydant et qu'elle possède un pH compris entre 3 et 11.

5. Procédé de teinture des fibres kératiniques et en particulier des fibres kératiniques humaines telles que les cheveux, caractérisé par le fait qu'il consiste à appliquer sur les fibres une composition de teinture (A) selon l'une quelconque des revendications 1 à 3, et à révéler la couleur en milieu alcalin, neutre ou acide à l'aide d'un agent oxydant qui est ajouté juste au moment de l'emploi à cette composition (A) ou qui est présent dans une composition (B) appliquée simultanément ou séquentiellement de façon séparée.

6. Dispositif à plusieurs compartiments ou "Kit" pour la teinture des fibres kératiniques et en particulier des fibres kératiniques humaines telles que les cheveux, caractérisé par le fait qu'il comporte au moins deux compartiments, dont l'un d'entre eux renferme une composition (A) telle que définie dans les revendications 1 à 3, et un autre une composition (B) comprenant un agent oxydant dans un milieu approprié pour la teinture.

7. Utilisation d'une composition de teinture selon l'une quelconque des revendications 1 à 4 ou d'un dispositif de teinture ou "Kit" à plusieurs compartiments selon la revendication 6, pour la teinture des fibres kératiniques humaines telles que les cheveux.

**Claims**

1. Oxidation dyeing composition for keratinous fibres, in particular for human keratinous fibres such as hair, of the type comprising, in a medium appropriate for dyeing, at least one oxidation dye precursor and at least one coupler, characterized in that it contains, as oxidation dye precursor, 2-(β-hydroxyethyl)-para-phenylenediamine and, as coupler, a combination of 2-methylresorcinol and 3-aminophenol, or the addition salts of these various compounds with an acid.

2. Dyeing composition according to Claim 1, characterized in that the addition salts with an acid are chosen from hydrochlorides, sulphates, hydrobromides and tartrates.

3. Dyeing composition according to either of Claims 1 and 2, characterized in that 2-(β-hydroxyethyl)-para-phenylenediamine or its salts is present in a concentration of between 0.01 and 10 % by weight with respect to the total weight of the composition and preferably between 0.05 and 5 % by weight, in that 2-methylresorcinol or its salts is present in a concentration of between 0.01 and 5 % by weight with respect to the total weight of the composition and preferably between 0.05 and 3 % by weight and in that 3-aminophenol or its salts is present in a concentration of between 0.01 and 5 % by weight with respect to the total weight of the composition and preferably between 0.05 and 3 % by weight.

4. Dyeing composition according to any one of Claims 1 to 3, which is ready-to-use, characterized in that it additionally contains an oxidizing agent and in that it has a pH of between 3 and 11.

5. Process for dyeing keratinous fibres and in particular human keratinous fibres such as hair, characterized in that it comprises applying to the fibres a dyeing composition (A) according to any one of Claims 1 to 3 and in developing the colour in alkaline, neutral or acidic medium using an oxidizing agent which is added to this composition (A) only at the time of use or which is present in a composition (B) separately applied simultaneously or sequentially.

6. Multi-compartment device or "kit" for dyeing keratinous fibres and in particular human keratinous fibres such as hair, characterized in that it contains at least two compartments, one of which contains a composition (A) such as defined in Claims 1 to 3 and another of which contains a composition (B) comprising an oxidizing agent in a medium appropriate for dyeing.

7. Use of a dyeing composition according to any one of Claims 1 to 4 or of a multi-compartment dyeing device or "kit" according to Claim 6 for dyeing human keratinous fibres such as hair.

**Patentansprüche**

1. Zusammensetzung zum oxidativen Färben von Keratinfasern, insbesondere von menschlichen Keratinfasern, wie Haaren, die in einem zum Färben geeigneten Medium mindestens ein Farbstoffvorprodukt der Oxidationsfarbe und mindestens einen Kuppler enthält,
dadurch gekennzeichnet, daß
sie 2-(β-Hydroxyethyl)-p-phenylendiamin als Farbstoffvorprodukt der Oxidationsfarbe und eine Kombination aus 2-Methylresorcin und 3-Aminophenol als Kuppler oder die Additionssalze dieser verschiedenen Verbindungen mit einer Säure enthält.

2. Zusammensetzung zum Färben mach Anspruch 1, dadurch gekennzeichnet, daß die Additionssalze mit einer Säure unter Hydrochloriden, Sulfaten, Hydrobromiden und Tartraten ausgewählt sind.

3. Zusammensetzung zum Färben nach einem der Ansprüche 1 oder 2, dadurch gekennzeichnet, daß 2-(β-Hydroxyethyl)-p-phenylendiamin oder seine Salze in einer Konzentration von 0,01 bis 10 Gew.-% und vorzugsweise von 0,05 bis 5 Gew.-%, bezogen auf das Gesamtgewicht der Zusammensetzung, 2-Methylresorcin oder seine Salze in einer Konzentration von 0,01 bis 5 Gew.-% und vorzugsweise im Bereich von 0,05 bis 3 Gew.-%, bezogen auf das Gesamtgewicht der Zusammensetzung, und 3-Aminophenol oder seine Salze in einer Konzentration von 0,01 bis 5 Gew.-% und vorzugsweise im Bereich von 0,05 bis 3 Gew.-%, bezogen auf das Gesamtgewicht der Zusammensetzung, vorliegen.

4. Zusammensetzung zum Färben nach einem der Ansprüche 1 bis 3, die anwendungsfertig ist, dadurch gekennzeichnet, daß sie ferner ein Oxidationsmittel enthält und einem pH-Wert von 3 bis 11 aufweist.

5. Verfahren zum Färben von Keratinfasern und insbesondere menschlichen Keratinfasern, wie Haaren, dadurch gekennzeichnet, daß es darin besteht, auf die Fasern eine Zusammensetzung zum Färben (A) nach einem der Ansprüche 1 bis 3 aufzutragen und die Farbe in alkalischem, neutralem oder saurem Medium mit einem Oxidationsmittel zu entwickeln, das der Zusammensetzung (A) bei der Anwendung zugesetzt wird oder das in einer Zusammensetzung (B) vorliegt, die gleichzeitig oder getrennt daran anschließend aufgetragen wird.

6. Vorrichtung mit mehreren Anteilungen oder "Kit" zum Färben von Keratinfasern und insbesondere menschlichen Keratinfasern, wie Haaren, dadurch gekennzeichnet, daß sie mindestens zwei Abteilungen aufweist, wobei eine Abteilung eine Zusammensetzung (A) nach den Ansprüchen 1 bis 3 und die andere eine Zusammensetzung (B) enthält, die ein Oxidationsmittel in einem zum Färben geeigneten Medium enthält.

7. Verwendung einer Zusammensetzung zum Färben nach einem der Ansprüche 1 bis 4 oder einer Vorrichtung zum Färben oder eines "Kits" mit mehreren Abteilungen nach Anspruch 6 zum Färben von menschlichen Keratinfasern, wie dem Haar.